# EUROPEAN PATENT SPECIFICATION

(11) **EP 2 239 001 B1**
(45) Date of publication and mention of the grant of the patent: **14.03.2012**
(21) Application number: 10158210.4
(22) Date of filing: 29.03.2010
(51) Int. Cl.: A61M 15/00

(54) **Dry powder inhaler device**
Trockenpulverinhalatorvorrichtung
Dispositif d'inhalateur en poudre sèche

(30) Priority: 30.03.2009 TR 200902446
(43) Date of publication of application: 13.10.2010
(73) Proprietor: Sanovel Ilac Sanayi ve Ticaret A.S., 34398 Istanbul (TR)
(72) Inventor: Toksöz, Ahmet, 34398, Istanbul (TR); Toksöz, Zafer, 34398, Istanbul (TR); Cifter, Ümit, 34398, Istanbul (TR)
(74) Representative: Sevinç, Erkan

(56) References cited:
- US-A1- 2002 032 409
- US-A1- 2005 017 017
- US-A1- 2005 154 491
- US-A1- 2006 196 504
- US-A1- 2009 078 252

## Description

### Technical Field of Invention

The present invention relates to a device for administering dry powder inhalation medicaments.

The present invention more particularly relates to developments conducted at the lock and blister advancement parts of dry powder inhaler devices.

### Background of Invention

Diseases such as asthma, bronchitis, and COLD (Chronic Obstructive Lung Disease) substantially decrease the quality of human life, despite the developments which have been carried out in the diagnosis and therapy thereof in recent years. It has been proposed to administer the medicaments via inhalers for optimizing the treatment of such diseases. The inhalation route of treatment is the most preferred one and it is expected to remain so, as the first option, in the future. The most important advantage of using medicaments via inhalation route is based on providing a more efficient therapy by making use of lesser medicaments, delivering higher concentration of medicaments to respiration channels, and particularly decreasing the systemic side effects of medicaments. The most important causes of the lack of a satisfactory control of patients albeit the presence of quite efficient treatments against respiratory tract diseases are stated to be as the noncompliance, arising from the inefficient use of inhalers and from inadequate compliance to the physician-recommended treatments.

Various inhaler devices for administering inhalation medicaments have been proposed until date. These devices are basically classified in two groups, i.e. metered dose inhalers and dry powder inhalers. These devices are structurally provided with basic components such as an actuator, counter, housing, lid, lock, etc. Additionally, powder inhalation medicaments are kept in reservoirs or containers such as blisters, capsules, etc. Blisters are structured from two basic parts, a main layer provided with cavities holding the medicament, and a strippable protective layer. Some of the currently-available dry powder inhaler devices are as follows.

EP0211595 discloses an inhaler device with a disk, which comprises a plurality of drug-carrying compartments. The drug-carrying compartments of disks are pierced by means of a needle and then served for use. This inhaler device comprises mountable and dismountable parts, whereas the replacement of drug-carrying disks is left to users. This type of embodiment leads to difficulties in use, leaving the hygiene control at an undesired level.

US 2009/078252 A1 discloses a fault indicator for a strip advancement mechanism comprising a base that defines a fault sensing portion of a path for at least a component of a strip, an element for bearing against a strip, or a component of the strip, while it passes through the fault sensing portion of the path, wherein the element is adapted to move from that bearing or non-fault position, to a fault indicating position in the event of the strip or the component of the strip ceasing to pass through the fault sensing portion of the path.

In another type of such devices, blisters are placed to a single container. Blisters are held with hand by their ends, the drug-containing part is constrained at a separate container and the blisters are stripped. Exposed drug is used by the user with the aid of a mouthpiece. Since no counter system is provided here, the consumed amount of drug is not known per se. Elpenhaler® may be referred to, to exemplify such type of embodiments.

In another type of inhalers, the capsule is placed to a single container. A needle provided in the device pierces the capsule, and the drug exposed is inhaled by the user. This type of device obviously brings the drawbacks of use difficulty, hygiene inadequacy, and forces the patients to carry excessive amount drugs with themselves.

A further type of current inhaler devices has the form of a disk and comprises multiple medicament compartments. These compartments contain medicament cartridges, the medicaments are made ready for use after piercing with the aid of a needle, and are administered by means of a mouthpiece. These devices further contain a disk-shaped counter, so that the number of consumed medicament cartridges is metered by means of said counter. WO2008114034, WO2005004962 may be cited herein to exemplify such type of devices. Such devices can take a determined number of medicaments into the disk, so that an increase in the device volume as result of increasing the number of medicaments therein gives rise to carrying/handling-, aesthetics- and use-related unpleasant outcomes. Additionally, they require the use of extra apparatuses for the counter.

The devices disclosed in patent applications WO9931952 and WO9947099 comprises blisters and are used by means of a separate mouthpiece. This type of devices also has drawbacks with respect to carrying, hygiene, and use.

Another type of inhaler comprises an opening lid and an arm advancing a blister mechanism in general terms. In order to drive said mechanism, the operation is terminated with at least two forward and backward movements along a disk-shaped body. The use reliability of device is ensured with an additional outer body. The use of extra devices, the necessity towards multistep operations, and the difficulties arising therefrom in such devices may cause to unwanted outcomes, such as wasting the drugs due to improper use.

Various types of lock mechanism have been developed so far to ensure operation reliability in currently-used inhaler devices. These embodiments have aimed at preventing the unnecessary use of drugs. The applications EP1774984, US2002170560 disclose lock embodiments used in inhaler devices.

The extent of the initial operational force to be applied to a button, arm, etc. to advance the contained blister and the extent of the stepwise advancement thereof in current blister inhaler devices are serious problems for the user. Particularly the case in which the user applies an inadequate force to this arm or button gives rise to a semi-opening of the blister, leading to an inadequate administration of the aimed dosage. On the contrary, an extreme force applied causes an excessive dosage to be released. In result, the need towards an embodiment which would ensure an accurate administration of the required amount of drug is obviously of vital importance.

The use of the current inhaler devices necessitates training and practice to some extent. The development of inhaler devices always occurs in the form of practical systems, providing the patients with improved convenience of use. Selecting the proper device for a respective patient is also an important issue. Many criteria, such as a patient's cognitive and physical efficiency, ease of use, reliability and price, etc. are considered while a device is selected.

In result, there is a need for development in the field of inhaler devices, providing high-accuracy operation, as well as advantages in terms of cost, volume, and usage.

### Objects and Brief Description of Invention

The present invention relates to an improved inhaler device for use with dry powder inhaling purposes, eliminating all aforesaid problems and bringing additional advantages to the relevant prior art.

Under the light of the prior art, the main object of the present invention is to provide a dry powder inhaler device, which can be operated at a desired accuracy, can peel off the blister for use, and can perform this process faultlessly as compared to precedent devices.

Another object of the present invention is to provide an inhaler device embodiment, which ensures a complete peeling-off of each medicament-carrying blister cavity by way of a lock mechanism having an actuating element transitionally displaceable along a predetermined distance.

A further object of the present invention is to realize a powder inhaler device provided with a mechanism, which ensures automatic setup of a subsequent device use, without requiring user intervention.

Still a further object of the present invention is to provide use safety, through a device which comprises a lock and lid mechanism operating synchronously and compatibly with the advancement mechanism.

In order to achieve all objects, referred to above and might be derived from the appended detailed description, novelty is made on a dry powder inhaler device, comprising an outer housing, an inner body disposed in the outer housing, a lid pivoted on the outer housing and closed by a rotational movement, a blister strip, a lower reservoir containing this strip in rolled form, a second additional gearwheel disposed on a left medial reservoir for rolling and storing the main layer of the blister comprising the medicament cavities, emerging after said blister strip is moved and separated into its layers, a right gear set disposed in the right medial reservoir for rolling and storing the protective layer of the blister strip, a left upper gearwheel proper for rolling and separating the blister strip, and a mouthpiece positioned just at the upper part of said left upper gearwheel.

In a preferred embodiment of the present invention, the subject inhaler device comprises an actuating element placed so as to be displaced axially in a slot disposed in the outer body, as well as a plurality of linear teeth, disposed over the actuating element for converting a linear motion imparted to the actuating element into rotational motion by means of an intermediate gearwheel to which it is engaged. The inhaler device comprises a locking clips, placed to undergo rotational motion around a pivot contact pin on the outer housing and set up to maintain its current position under the effect of a support element, and said locking clips having a locking lug at its one end. The actuating element is provided with a retaining lug, which is positioned so as to engage the corresponding lug over the actuating element for locking the actuating element at the fully-pushed position, when it is pushed so. There are provided a spring, which is placed so as to become loaded when the actuating element is pushed towards the interior of the slot, and which allows the actuating element to come out when it is released, as well as a push arm, which is integrated to the lid and when the lid is closed to full extent, which allows the actuating element by way of exerting pressure to that end of the locking clips which does not contain the locking lug.

In a preferred embodiment of the present invention, there is provided a left lower gearwheel as part of the left gear set disposed in the upper reservoir which gearwheel engages the intermediate gearwheel, by which the any linear motion applied to said actuating element is converted to rotational motion, and which gearwheel transfers the rotational motion imparted from said intermediate gearwheel to the right gear set, and to the second additional gearwheel by means of the first additional gearwheel.

In a preferred embodiment of the present invention, at least one angular lug is provided on the left lower gearwheel to let the left gear set rotate in single-direction

According to a preferred embodiment of the present invention, the right gear set is composed of at least one angular lug element transmitting the single-direction rotational motion thereof, and a right medial gearwheel containing at least one retaining lug retaining the blister, and a right upper gearwheel, over which the protective layer of the blister strip is rolled, and a right lower gearwheel driven by the left lower gearwheel.

In a preferred embodiment of the present invention, there is provided a blister retaining lug, integrated to said second additional gearwheel, and fastening the end of the main blister layer to said second additional gearwheel.

According to a preferred embodiment, the present invention comprises at least one guide means positioned on the inner surface of the outer housing, and at least one corresponding guide element guided to the guide means on the outer surface of the actuating element, for providing the stroke path control while the actuating element is displaced in the slot.

According to a preferred embodiment, the present invention comprises a distance adjustment piece positioned on the outer housing for preventing said actuating element from coming out of the slot during its transitional movement.

In a preferred embodiment of the present invention, there is provided at least one circular displacement surface with a curved form on the lower surface of the locking clips, for allowing the actuating element to conduct axial displacement on the lower surface of the locking clips.

In a preferred embodiment of the present invention, there is provided at least one locking arm integrated to the inner part of the lid, for locking the operating mechanism of the subject device by engaging in between the teeth of the right lower gearwheel, when the lid is closed to full extent.

In a preferred embodiment of the present invention, there is provided at least one opening on the outer housing for allowing the locking arm engage the in between teeth of the right lower gearwheel.

In a preferred embodiment of the present invention, the spring is V-shaped, said spring becoming loaded with the actuating element pushed into the reservoir, and causing the actuating element come out when the same is released.

Structural and characteristic features, and all advantages of the present invention shall be made clear by means of annexed figures described here below and a detailed description written with references to said figures; therefore the present invention must be evaluated by taking into consideration these figures and the detailed description.

### Brief Description of Figures

Figure 1 is an exploded schematic view of an illustrative embodiment according to the present invention.
Figure 2 is a perspective schematic view of the inner body according to the present invention.

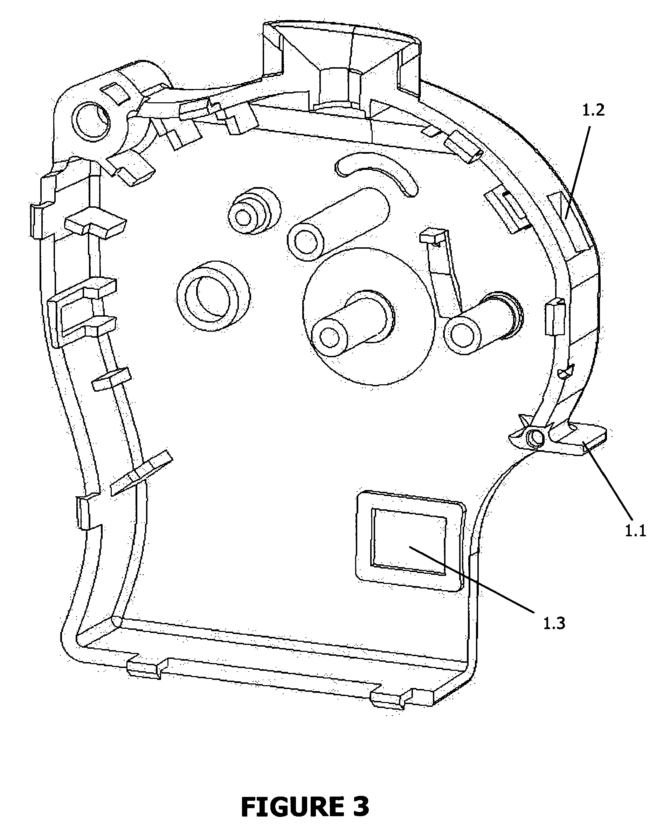

Figure 3 is a perspective schematic view of the outer housing according to the present invention.
Figure 4 is an illustrative schematic view of the actuating element and locking clips according to the present invention.

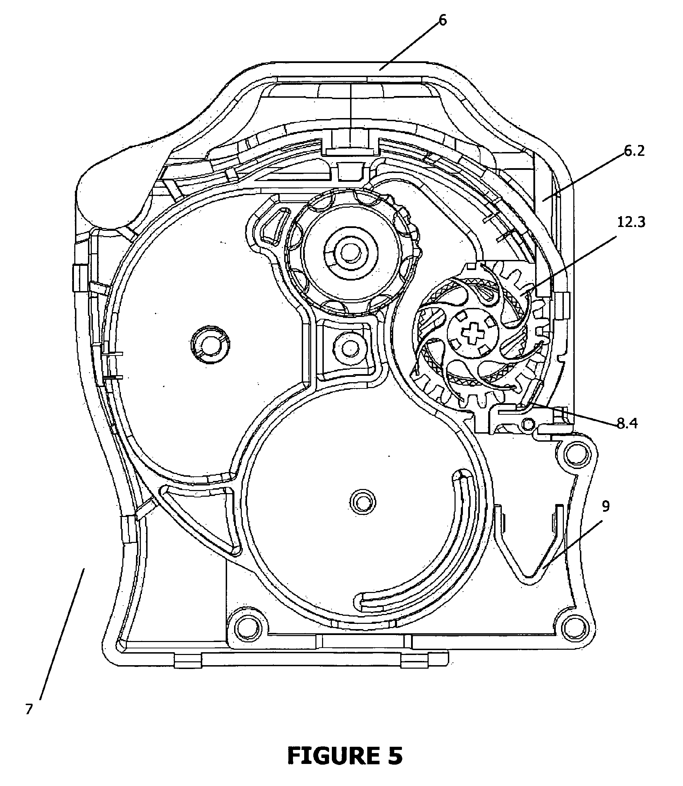

Figure 5 is a cross-sectional schematic view of the subject inhaler device with the lid in closed position.
Figure 6 is an illustrative schematic view of the interior of the lid according to the present invention.
Figure 7 is an exploded schematic view of the left gear set according to the present invention.
Figure 8 is an exploded schematic view of the right gear set according to the present invention.

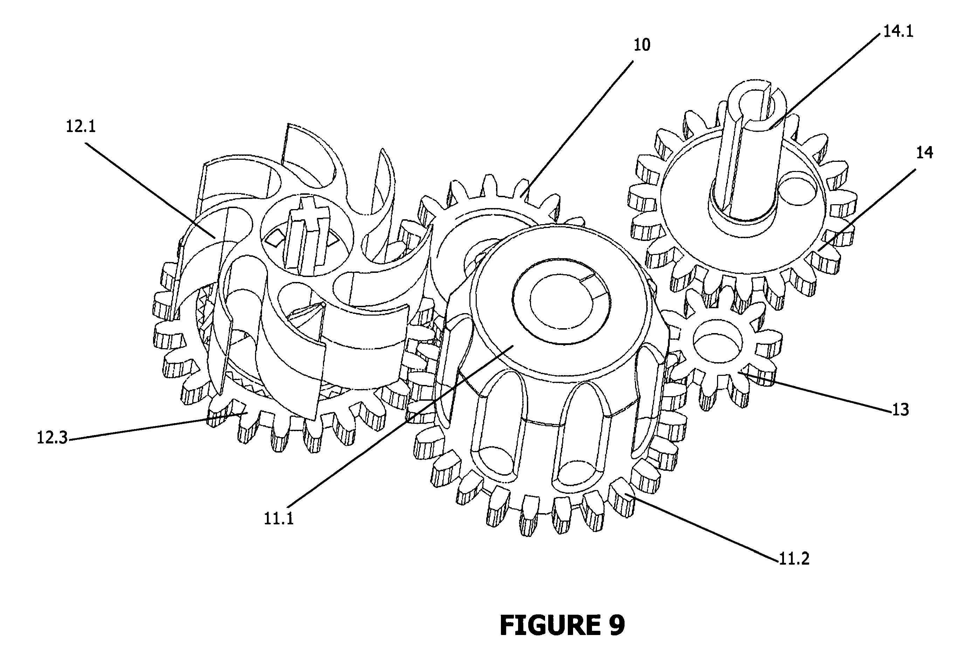

Figure 9 is a perspective schematic view of the gearwheels used in the drive transmission mechanism according to the present invention, illustrating the mutual engagements thereof.

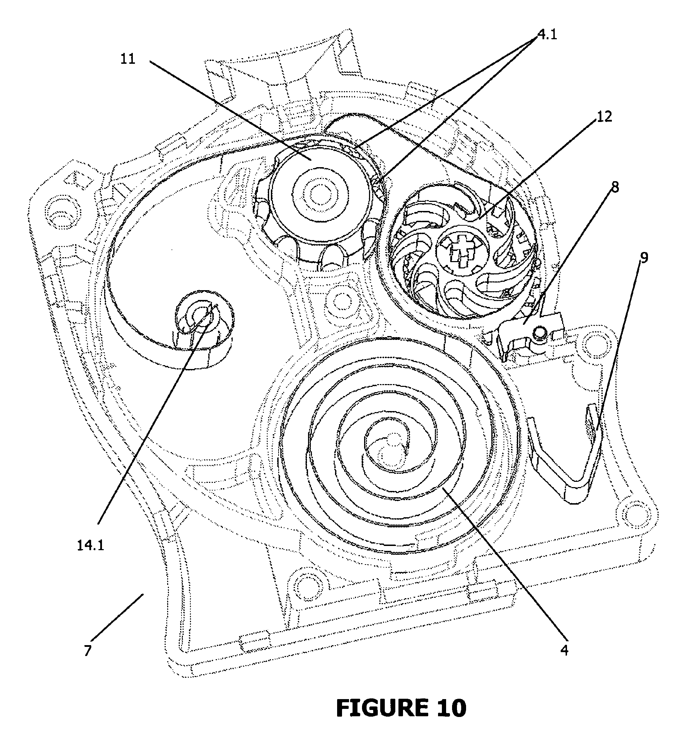

Figure 10 is a cross-sectional schematic view of the subject inhaler device with a blister inserted and the actuating element not pushed in.

### Reference Numbers in Figures

1. Outer housing
1.1 Distance adjustment piece
1.2 Opening
1.3 Guide
1.4 Slot
2. Inner body
2.1 Inner body pin
2.2 Lower reservoir
2.3 Left medial reservoir
2.4 Right medial reservoir
2.5 Upper reservoir
3. Mouthpiece
4. Blister
4.1 Cavity
5. Actuating element
5.1 Retaining lug
5.2 Linear teeth
5.3 Grip surface
5.4 Pin
5.5 Guide element
6. Lid
6.1 Push arm
6.2 Locking arm
7. Inhaler device
8. Locking clips
8.1 Lug
8.2 Contact pin
8.3 Displacement surface
8.4 Support element
9. Spring
9.1 Spring contact hole
10. Intermediate gearwheel
11. Left gear set
11.1 Left upper gearwheel
11.2 Left lower gearwheel
11.2.1 Angular lug
12. Right gear set
12.1 Right upper gearwheel
12.2 Right medial gearwheel
12.2.1 Medial gearwheel angular lug
12.2.2 Medial gearwheel retaining lug
12.3 Right lower gearwheel
13. First additional gearwheel
14. Second additional gearwheel
14.1 Blister retaining lug

### Detailed Description of the Invention

In the following detailed description, the inhaler device (7) according to the present invention shall be described illustratively by making references to annexed figures, only to make it clear without imposing any restrictions thereon.

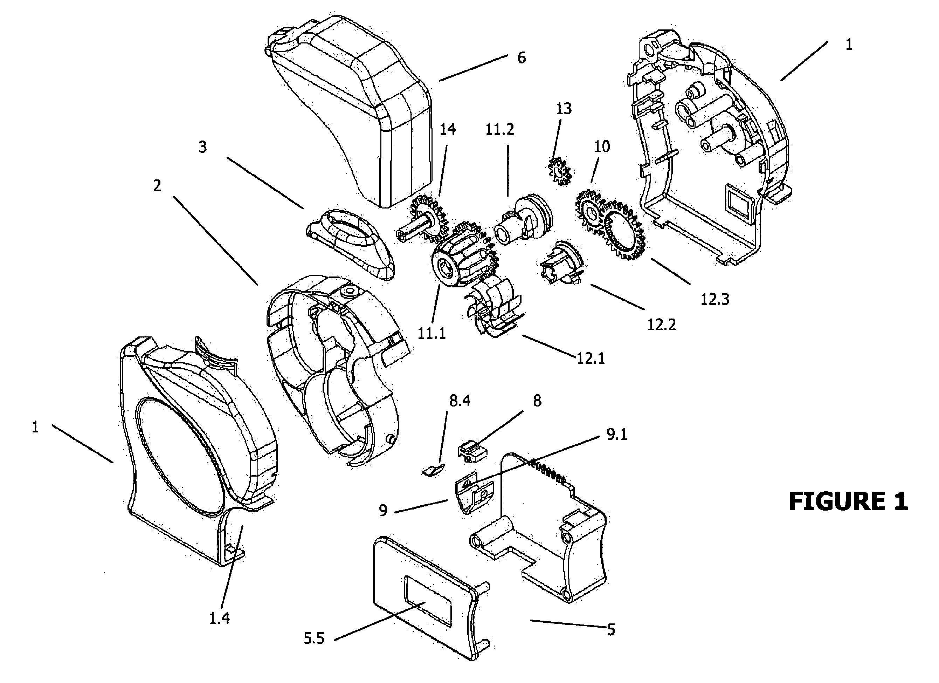

The outer housing (1) of the inhaler device (7) according to the present invention as illustrated in an exploded manner in Figure 1 is obtained by attaching two compatible parts to each other. The inner periphery of said parts comprises both fastening tabs for fastening the parts together, and reservoirs and pins, allowing the placement of the left and right gear set (11, 12) in the outer housing (1). When these two parts are gathered, a distance adjustment piece (1.1) is formed on the front medial part of the body and an opening (1.2) on the upper part. A mouthpiece (3) with a small medicament releasing opening is provided on the upper part of the body, whereas mutually-facing guide means (1.3) are formed in the interior of the body to guide the actuating element's (5) transitional movement, and a slot (1.4) is provided at the lower part of the body to provide a stroke path for the actuating element.

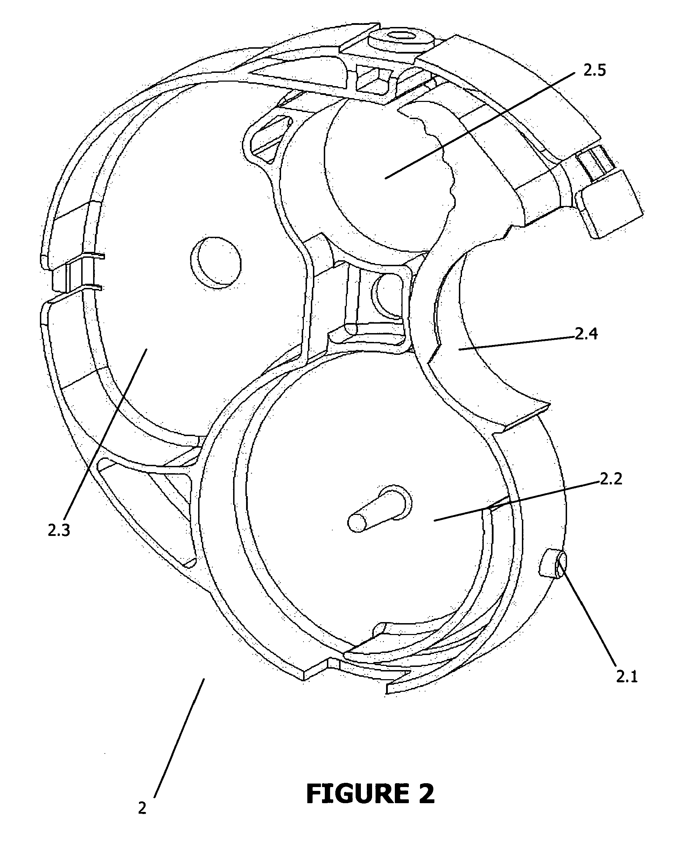

An inner body (2) is positioned in the interior of the outer housing (1) to accommodate the respective device components, as perspectively illustrated in Figure 2. This single-piece body (2) comprises lower, left and right medial and upper reservoirs (2.2, 2.3, 2.4, 2.5), respectively, along with recessed surfaces and lugs. An unused blister (4) is stored in the lower reservoir (2.2), this strip-formed blister (4) being extended along intermediate channels and subsequently separated into two parts, i.e. a main layer and a protective layer peeled off by means of the mechanism. The main layer, of which the end is fastened to the additional gearwheel retaining lug (14.1) is stored in the left medial reservoir (2.3) where the second additional gearwheel (14) is disposed, whereas one end of the protective layer is fastened to the medial gearwheel retaining lug (12.2.2) and wound to the right gear set (12).

At the beginning of the blister (4) advancing mechanism is provided with an actuating element (5), pushed or slid into the lower lateral side of said outer housing (1). The upper surface of the actuating element (5) is provided with a retaining lug (5.1) and preferably with a plurality of linear teeth (5.2). The actuating element (5) can be axially displaced in the slot (1.4) to an extent, which is determined by means of a guide element (5.5), guide means (1.3), and distance adjustment piece (1.1) provided on the body (1). The grip surface (5.3) provided on the exterior of the actuating element, by which a user exerts force to push said actuating element (5), is formed in an incurved manner to provide ease of use. There is provided a spring (9), disposed between the actuating element (5) and the lower reservoir (2.2), said spring becoming compressed (i.e. loaded), while the actuating element (5) is pushed into the slot (1.4). This spring (9) is preferably fastened by means of contact holes (9.1), which cooperate with a pin (5.4) provided at the rear of the interior of the actuating element (5) and with an inner body pin (2.1) disposed at the outer surface of the lower reservoir (2.2) facing the actuating element (5).

Any axial drive exerted to the linear teeth (5.2) on the actuating element (5) is first transmitted to the left lower gearwheel (11.2) of the left gear set (11) by means of an intermediate gearwheel (10) in the form of rotational motion. The rotational motion transmitted to said left lower gearwheel (11.2) both rotates the left gear set (11), and transmits this motion to the right lower gearwheel (12.3), so as to ensure the rotation of the right gear set (12) and the rotation of the second additional gearwheel (14) by means of the first additional gearwheel (13).

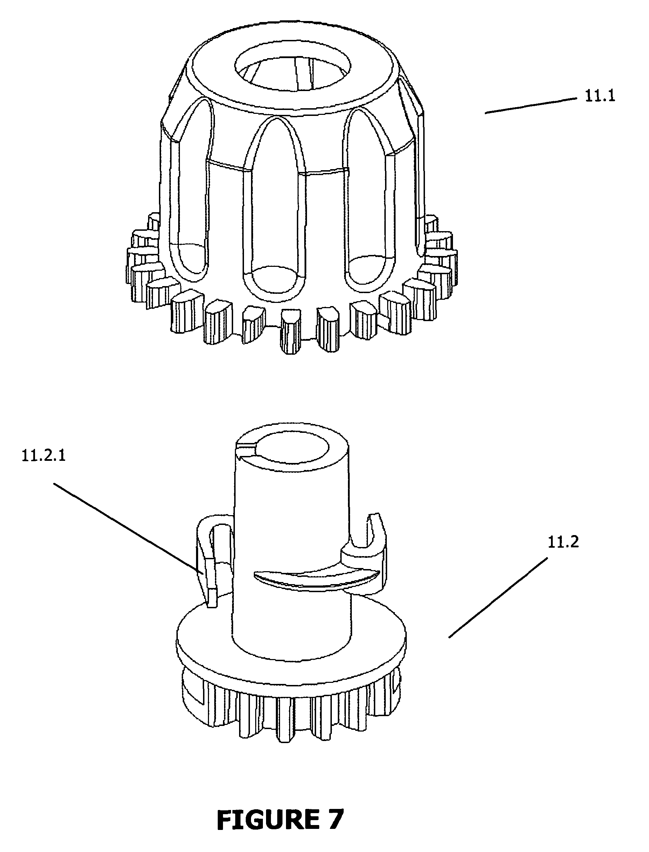

In order to provide said gearwheels (11) with single-direction rotation only, the left gear set (11) is composed of a left lower gearwheel (11.2) provided with angular lug element (11.2.1), and of a left upper gearwheel (11.1), executing rotational motion together with the left lower gearwheel (11.2) to displace the blister strip (4), as illustrated in detail in Figure 7.

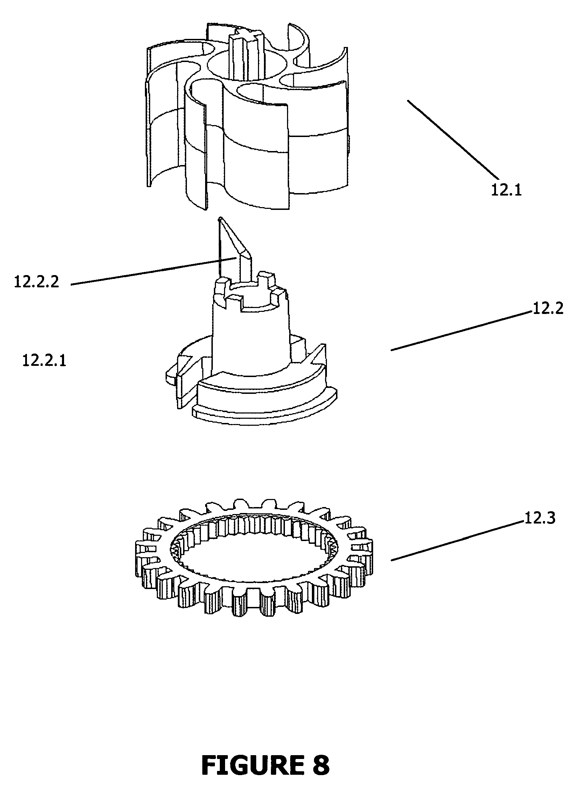

As illustrated in Figure 8, the right gear set (12) comprises a right lower gearwheel (12.3), which receives rotational drive from the left lower gearwheel and transfers the same to the right gearwheel set (12), an angular lug element (12.2.1) which provides said gearwheels (12) with single-direction rotational motion only, a right medial gearwheel (12.2) comprising a retaining lug (12.2.2) to fasten one end of the blister, and a right upper gearwheel (12.1), over which the protective layer of the blister strip (4) is rolled.

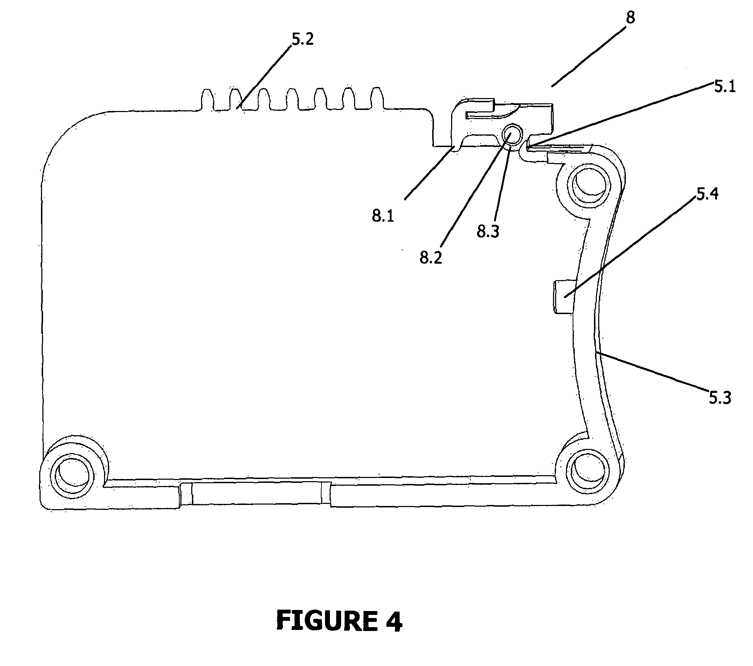

A locking mechanism is provided on the upper part of the actuating element (5), as illustrated in Figure 4. The clips (8) disposed here is in connection with the outer housing (1) by means of a pin (8.2), this connection being embodied so as to allow the pin to make a lever movement up- and downward. The clips (8) comprises an out-curved displacement surface (8.3) at its lower part, and a lug (8.1) at its tip. On the other hand, a support element (8.4) is positioned on the gap provided on the upper part of the clips, to let the clips maintain its current state at the open or closed position.

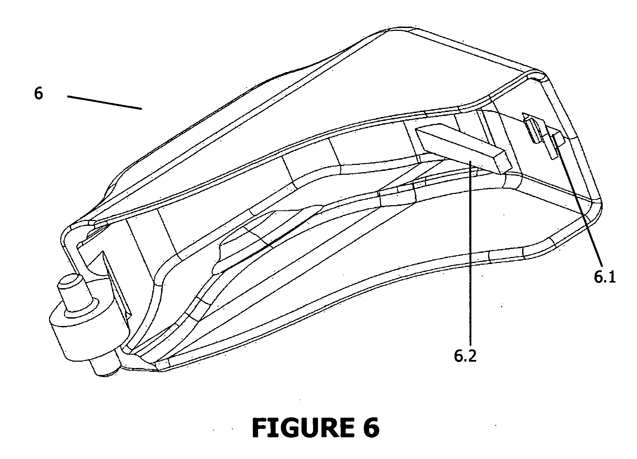

As illustrated in Figure 6, the inner part of the lid (6) is provided with a push arm (6.1) at the front, and a locking arm (6.2) at the rear. This push arm (6.1) contacts the locking clips (8) when the lid (6) is closed. At the same time the locking arm (6.2) passes through within the opening (1.2) at the outer housing and contacts the right gear set (12). In this case, while the locking arm (6.2) in contact with the right gear set (12) prevents the gear set (12) from moving, the push arm (6.1) exerts force onto the end of the locking clips that is not provided with the locking lug (8.1).

According to the details given above, the operation of the device according to the present invention is as follows. Following the opening of lid (6), a force is exerted by the user to the grip surface (5.3) of the actuating element (5). Then the actuating element (5) is guided by means of the guides (1.3, 5.5) provided in the body (1) and on itself and is slid in the slot (1.4). The linear teeth (5.2) provided on the top of the actuating element (5) transmits this drive to the intermediate gearwheel (10). The intermediate gearwheel (10) transfers this drive to the left lower gearwheel (11.2) and then to the left gear set (11) and to the first and second additional gearwheels (13, 14). Then the left and right gear sets (11, 12) execute rotational motion in opposite directions. The right gear set (12) is rotated clockwise, while the left gear set (11) is rotated counterclockwise. The blister (4) in contact with these gearwheels is advanced in the channels of the inner body (2), is passed through between the left and right gear sets (11, 12), and the medicament released from the blister is forced to the mouthpiece (3). The main layer of the blister, now separated into two parts, is passed through the left gear set (11) and rolled in the left medial reservoir (2.3), whereas the protective layer of the blister is rolled in the right gear set (12).

As a result of sliding the actuating element (5) into the interior of the outer housing (1), the retaining lug (5.1) provided on the actuating element (5) is coupled and fastened to the locking clips (8) lug (8.1) provided just over itself, resulting in the administration of a single dose of medicament. Keeping this slide-in action until the locking position is achieved, guarantees the complete peeling-off of the blister and ensures the accurate administration of the required dosage amount. As a result of this locking effect, the actuating element (5) becomes retained and it remains out-of-use for a short period of time. This slide-in action also causes the V-shaped spring (9) to become compressed between the actuating element's pin (5.4) and the inner body's pin (2.1).

After the user inhales the powder medicament, he/she closes the lid (6), so that the push arm (6.1) provided in the interior of the lid exerts a force to the rear part of the locking clips (8), the end thereof is lifted above, and the clips lug (8.1) and the retaining (5.1) are detached from each other. As a result of this, the V-shaped spring (9) in compressed form pushes back the actuating element (5) and restores the device for the next use, without requiring any user intervention. While the actuating element (5) is displaced backward, however, the mechanism does not move the blister backward. The one way angular lugs (11.2.1, 12.2.1) provided on the left lower gearwheel and right medial gearwheel (11.2, 12.2) prevent the mechanism from moving backward. The backward advancement is restrained by means of these angular lugs. Here, the movement of the actuating element (5), as it is released backward, is compensated by the left and right lower gearwheels (11.2, 12.3) independently from the gearwheels disposed over themselves by means of rotating backward. When the lid is closed, the locking arm (6.2) provided in the interior of the lid contacts the right gear set (12), so as to completely stop the mechanism as long as the lid is kept closed. The locking clips (8) is secured while it is at upper position by means of the support element (8.4) provided on the upper part thereof (8). It can be seen from the disclosure above, that the device according to the present invention can be operated by a simple single push action of the user. When the lid (6) is closed, the device (7) is set up automatically and is restored for the next use.

In result, an inhaler device is obtained with the embodiment disclosed above, this device being extremely accurate, reliably-operative, and cost- and volume-effective.

The design of components used may be varied in alternative embodiments according to the type of device being produced. In result, the protection scope of the present invention is set forth in appended Claims and cannot be restricted to the illustrative examples given above, under the detailed description. It is obvious that a person skilled in the relevant art can produce similar embodiments under the light of the foregoing disclosures, without departing from the main principles of the present invention.

## Claims

1. A dry powder inhaler device, comprising an outer housing (1), an inner body (2) disposed in the outer housing, a lid (6) pivoted on the outer housing (1) and closed by a rotational movement, a blister strip (4), a first reservoir (2.2) containing this strip in rolled form, a second additional gearwheel (14) disposed on a left medial reservoir (2.3) for rolling and storing the main layer of the blister which is moved and separated into its layers and which comprises a plurality of cavities (4.1) a right gear set (12) disposed in the right medial reservoir (2.4) for rolling and storing the protective layer of the blister strip, a first gearwheel (11.1) proper for rolling and separating the blister strip, and a mouthpiece (3) positioned just at the upper part of said first gearwheel, **characterized in that** the device further comprises;
an actuating element (5) placed so as to be displaced axially in a slot (1.4) disposed in the outer body (1),
a plurality of linear teeth (5.2), disposed over the actuating element (5) for converting a linear motion imparted to the actuating element into rotational motion by means of an intermediate gearwheel (10) to which it is engaged,
a locking clips (8), placed properly to undergo rotational motion around a pivot contact pin (8.2) on the outer housing, and set up to maintain its position under the effect of a support element (8.4), and said locking clips (8) having a locking lug (8.1) at its one end,
a retaining lug (5.1), which is positioned so as to engage the corresponding lug (8.1) on the actuating element (5) for locking the actuating element at and when the actuating element is in fully-pushed position,
a spring (9), which is placed so as to become compressed when the actuating element is pushed towards the interior of the slot (1.4), and which allows the actuating element to come out when it is released, and
a push arm (6.1), which is integrated to the lid (6), and which releases the actuating element (5) by way of exerting pressure to that end of the locking clips (8) which does not contain the locking lug (8.1).

2. A dry powder inhaler device (7) according to Claim 1, **characterized by** comprising a second gearwheel (11.2) as part of the left gear set (11) disposed in a second reservoir (2.5), which gearwheel (11.2) engages the intermediate gearwheel (10), by which the linear motion applied to said actuating element (5) is converted to rotational motion, and which gearwheel (11.2) transfers the rotational motion received from said intermediate gearwheel (10) to the right gear set (12), and to the second additional gearwheel (14) by means of the first additional gearwheel (13).

3. A dry powder inhaler device (7) according to claims 1 and 2, **characterized by** comprising at least one angular lug element (11.2.1) on the second gearwheel (11.2) for restricting let the left gear set (11) conduct single-direction rotational motion.

4. A dry powder inhaler device (7) according to claims 1 and 3, **characterized in that** the right gear set (12) is composed of at least one angular lug element (12.2.1) providing the single-direction rotational motion thereof, and a right medial gearwheel (12.2) containing at least one retaining lug (12.2.2) retaining the blister, and a third gearwheel (12.1), over which the protective layer of the blister strip is rolled, and a fourth gearwheel (12.3) driven by the second gearwheel (11.2).

5. A dry powder inhaler device (7) according to Claim 1, **characterized by** comprising a blister retaining lug (14.1), integrated to said second additional gearwheel (14), and fastening the end of the main blister layer to said second additional gearwheel (14).

6. A dry powder inhaler device (7) according to Claim 1, **characterized by** comprising at least one guide means (1.3) positioned on the inner surface of the outer housing (1), and at least one corresponding guide element (5.5) guided to the guide means (1.3) on the outer surface of the actuating element (5) for providing the stroke path control while the actuating element is displaced in the slot (1.4).

7. A dry powder inhaler device (7) according to Claim 1, **characterized by** comprising a distance adjustment piece (1.1) positioned on the outer housing (1) for preventing said actuating element (5) from coming out of the slot (1.4) during its transitional movement.

8. A dry powder inhaler device (7) according to Claim 1, **characterized by** comprising at least one circular displacement surface (8.3) with a curved form on the lower surface of the locking clips (8), for allowing the actuating element (5) to conduct axial displacement on the lower surface of the locking clips (8).

9. A dry powder inhaler device (7) according to claims 1 and 4, **characterized by** comprising at least one locking arm (6.2) integrated to the inner part of the lid (6), for locking the operating mechanism of the device (7) by engaging in between the teeth of the fourth gearwheel (12.3) when the lid is closed to full extent.

10. A dry powder inhaler device (7) according to claims 1 and 9, **characterized by** comprising at least one opening (1.2) on the outer housing (1), for allowing the locking arm (6.2) engage in between the teeth of the fourth gearwheel (12.3).

11. A dry powder inhaler device (7) according to Claim 1, **characterized in that** the spring (9) is V-shaped, said spring becoming loaded with the actuating element (5) pushed into the slot (1.4), and causing the actuating element (5) to come out when the same is released.

## Patentansprüche

1. Trockenpulverinhalatorvorrichtung, umfassend ein äußeres Gehäuse (1), einen inneren Körper (2), der in dem äußeren Gehäuse angeordnet ist, einen Deckel (6), der schwenkbar an dem äußeren Gehäuse (1) gelagert ist und durch eine Drehbewegung geschlossen wird, einen Blisterstreifen (4), ein erstes Reservoir (2.2), das diesen Streifen in gerollter Form enthält, ein zweites zusätzliches Zahnrad (14), das an einem linken mittleren Reservoir (2.3) zum Rollen und Lagern der Hauptschicht des Blisters angeordnet ist, welcher bewegt wird und in seine Schichten zerlegt wird und welcher eine Vielzahl Hohlräume (4.1) aufweist, einen rechten Zahnradsatz (12), der in dem rechten mittleren Reservoir (2.4) zum Rollen und Lagern der Schutzschicht des Blisterstreifens angeordnet ist, ein erstes Zahnrad (11.1) geeignet zum Rollen und Zerlegen des Blisterstreifens, und ein Mundstück (3), das direkt an dem oberen Teil des ersten Zahnrads angeordnet ist, **dadurch gekennzeichnet, dass** die Vorrichtung weiter umfasst;
ein Betätigungselement (5), das so angeordnet ist, dass es axial in einen Einschub (1.4), der in dem äußeren Gehäuse (1) angeordnet ist, verschoben wird,
eine Vielzahl von linearen Zähnen (5.2), die über dem Betätigungselement (5) angeordnet sind, um eine lineare Bewegung, die dem Betätigungselement vermittelt wird, in eine Drehbewegung mittels eines dazwischenliegenden Zahnrads (10), in welches es greift, zu konvertieren,
eine Verriegelungsklemme (8), die geeignet angeordnet ist, um eine Drehbewegung um einen Gelenkkontaktstift (8.2) an dem äußeren Gehäuse auszuführen, und die eingerichtet ist, um ihre Position unter der Einwirkung eines Trägerelements (8.4) aufrechtzuerhalten, wobei die Verriegelungsklemme (8) eine Schließnase (8.1) an ihrem einen Ende hat,
eine Haltenase (5.1), welche so positioniert ist, dass sie in Eingriff mit der entsprechenden Nase (8.1) an dem Betätigungselement (5) steht, um das Betätigungselement zu verriegeln bei und wenn das Betätigungselement in voll gedrückter Position ist,
eine Feder (9), welche so angeordnet ist, dass sie zusammengedrückt wird, wenn das Betätigungselement in Richtung des Inneren des Einschubs (1.4) gedrückt wird, und welche dem Betätigungselement erlaubt herauszukommen, wenn es gelöst wird, und
einen Schubarm (6.1), welcher in den Deckel (6) integriert ist, und welcher das Betätigungselement (5) durch Ausüben von Druck auf dasjenige Ende der Verriegelungsklemme (8) löst, welches die Schließnase (8.1) nicht umfasst.

2. Trockenpulverinhalatorvorrichtung (7) nach Anspruch 1, **dadurch gekennzeichnet, dass** sie ein zweites Zahnrad (11.2) als Teil des linken Zahnradsatzes (11) umfasst, der in einem zweiten Reservoir (2.5) angeordnet ist, wobei dieses Zahnrad (11.2) in das dazwischenliegende Zahnrad (10) greift, durch welches die lineare Bewegung, die auf das Betätigungselement (5) angewendet wird, in eine Drehbewegung konvertiert wird, und wobei dieses Zahnrad (11.2) die Drehbewegung, die es von dem dazwischenliegenden Zahnrad (10) empfängt, auf den rechten Zahnradsatz (12) und auf das zweite zusätzliche Zahnrad (14) mittels des ersten zusätzlichen Zahnrads (13) überträgt.

3. Trockenpulverinhalatorvorrichtung (7) nach Anspruch 1 und 2, **dadurch gekennzeichnet, dass** sie mindestens ein winkelförmiges Nasenelement (11.2.1) an dem zweiten Zahnrad (11.2) umfasst, um den linken Zahnradsatz (11) darauf zu beschränken, eine Einrichtungsdrehbewegung durchzuführen.

4. Trockenpulverinhalatorvorrichtung (7) nach den Ansprüchen 1 und 3, **dadurch gekennzeichnet, dass** der rechte Zahnradsatz (12) vom mindestens einem winkelförmigen Nasenelement (12.2.1) gebildet ist, das für die Einrichtungsdrehbewegung hiervon sorgt, und einem rechten mittleren Zahnrad (12.2), das mindestens eine Haltenase (12.2.2) umfasst, die den Blister hält, und einem dritten Zahnrad (12.1), über welches die Schutzschicht des Blisterstreifens gerollt ist, und einem vierten Zahnrad (12.3), das durch das zweite Zahnrad (11.2) angetrieben wird.

5. Trockenpulverinhalatorvorrichtung (7) nach Anspruch 1, **dadurch gekennzeichnet, dass** sie eine Blisterhaltenase (14.1) umfasst, die in das zweite zusätzliche Zahnrad (14) integriert ist und das Ende der Hauptblisterschicht an dem zweiten zusätzlichen Zahnrad (14) befestigt.

6. Trockenpulverinhalatorvorrichtung (7) nach Anspruch 1, **dadurch gekennzeichnet, dass** sie mindestens eine Führungseinrichtung (1.3) umfasst, die an der inneren Oberfläche des äußeren Gehäuses (1) positioniert ist, und mindestens ein entsprechendes Führungselement (5.5), das zu der Führungseinrichtung (1.3) an der äußeren Oberfläche des Betätigungselements (5) geführt wird, um für die Hubwegkontrolle zu sorgen, während das Betätigungselement in den Einschub (1.4) verschoben wird.

7. Trockenpulverinhalatorvorrichtung (7) nach Anspruch 1, **dadurch gekennzeichnet, dass** sie ein Abstandanpassungsstück (1.1) umfasst, das an dem äußeren Gehäuse (1) positioniert ist, um zu verhindern, dass das Betätigungselement (5) aus dem Einschub (1.4) während seiner Übergangsbewegung herauskommt.

8. Trockenpulverinhalatorvorrichtung (7) nach Anspruch 1, **dadurch gekennzeichnet, dass** sie mindestens eine kreisförmige Verschiebeoberfläche (8.3) mit einer gewölbten Form an der unteren Oberfläche der Verriegelungsklemme (8) umfasst, um dem Betätigungselement (5) zu erlauben, eine axiale Verschiebung an der unteren Oberfläche der Verriegelungsklemme (8) durchzuführen.

9. Trockenpulverinhalatorvorrichtung (7) nach den Ansprüchen 1 und 4, **dadurch gekennzeichnet, dass** sie mindestens einen Verriegelungsarm (6.2) umfasst, der in den inneren Teil des Deckels (6) integriert ist, um den Betriebsmechanismus der Vorrichtung (7) durch Greifen zwischen die Zähne des vierten Zahnrads (12.3) zu sperren, wenn der Deckel in vollem Umfang geschlossen ist.

10. Trockenpulverinhalatorvorrichtung (7) nach den Ansprüchen 1 und 9, **dadurch gekennzeichnet, dass** sie mindestens eine Öffnung (1.2) an dem äußeren Gehäuse (1) umfasst, um dem Verriegelungsarm (6.2) zu erlauben, zwischen die Zähne des vierten Zahnrads (12.3) zu greifen.

11. Trockenpulverinhalatorvorrichtung (7) nach Anspruch 1, **dadurch gekennzeichnet, dass** die Feder (9) V-förmig ist, wobei die Feder mit dem Betätigungselement (5), das in den Einschub (1.4) gedrückt wird, belastet wird und verursacht, dass das Betätigungselement (5) herauskommt, wenn dasselbe gelöst wird.

## Revendications

1. Dispositif inhalateur de poudre sèche, qui comprend un boîtier extérieur (1), un corps intérieur (2) disposé dans le boîtier extérieur, un couvercle (6) monté pivotant sur le boîtier extérieur (1) et fermé par un mouvement de rotation, un ruban d'emballage pelliculé (4), un premier réceptacle (2.2) contenant ce ruban sous une forme enroulée, une seconde roue dentée supplémentaire (14) disposée dans un réceptacle central gauche (2.3) destiné à l'enroulement et à la conservation de la couche principale du pelliculage qui est déplacé et séparé en ses couches, et qui comprend plusieurs cavités (4.1), un groupe droit d'engrenage (12) disposé dans un réceptacle central droit (2.4) destiné à l'enroulement et à la conservation de la couche protectrice du ruban d'emballage pelliculé, une première roue dentée (11.1) convenant pour l'enroulement et la séparation du ruban d'emballage pelliculé, et une embouchure (3) située juste à la partie supérieure de ladite première roue dentée, **caractérisé en ce que** le dispositif comprend en outré :
un élément d'actionnement (5) placé de façon à être déplacé axialement dans une fente (1.4) ménagée dans le boîtier extérieur (1),
plusieurs dents à disposition rectiligne (5.2), situées à la partie supérieure de l'élément d'actionnement (5) pour convertir un déplacement rectiligne imparti à l'élément d'actionnement en un déplacement rotatif au moyen d'une roue dentée intermédiaire (10) avec laquelle elles viennent en prise,
un élément éclipsable de verrouillage (8), placé d'une manière convenable pour être soumis à un déplacement rotatif autour d'un doigt de contact (8.2) formant pivot sur le boîtier extérieur, et disposé de façon à maintenir sa position sous l'effet d'un élément de support (8.4), et ledit élément éclipsable de verrouillage (8) présentant une patte de verrouillage (8.1) à sa première extrémité,
une patte de retenue (5.1), qui est disposée de façon à venir en prise sur la patte correspondante (8.1) située sur l'élément d'actionnement (5) pour verrouiller l'élément d'actionnement à l'endroit où et lorsque l'élément d'actionnement est dans sa position totalement poussée,
un ressort (9), qui est placé de façon à se trouver comprimé lorsque l'élément d'actionnement est poussé vers l'intérieur de la fente (1.4), et qui permet à l'élément d'actionnement de sortir lorsqu'il est détendu, et
un doigt de poussée (6.1) qui est intégré au couvercle (6), et qui relâche l'élément d'actionnement (5) en exerçant une pression sur l'extrémité de l'élément éclipsable de verrouillage (8) qui ne comporte pas la patte de verrouillage (8.1).

2. Dispositif inhalateur de poudre sèche (7) suivant la revendication 1, **caractérisé en ce qu'**il comprend une seconde roue dentée (11.2) faisant partie d'un groupe gauche d'engrenage (11) disposé dans un second réceptacle (2.5), laquelle roue dentée (11.2) vient en prise avec la roue dentée intermédiaire (10), au moyen de laquelle le déplacement rectiligne appliqué audit élément d'actionnement (5) est converti en déplacement rotatif, et laquelle roue dentée (11.2) transfère le déplacement rotatif reçu de ladite roue dentée intermédiaire (10) au groupe droit d'engrenage (12) et à la seconde roue dentée supplémentaire (14) au moyen d'une première roue dentée supplémentaire (13).

3. Dispositif inhalateur de poudre sèche (7) suivant les revendications 1 et 2, **caractérisé en ce qu'**il comprend au moins un élément en forme de patte angulaire (11.2.1) sur la seconde roue dentée (11.2) en vue d'une limitation laissant le groupe gauche d'engrenage (11) suivre un déplacement rotatif dans un seul sens.

4. Dispositif inhalateur de poudre sèche (7) suivant les revendications 1 et 3, **caractérisé en ce que** le groupe droit d'engrenage (12) est composé d'au moins un élément en forme de patte angulaire (12.2.1), assurant le déplacement rotatif dans un seul sens de celui-ci, et d'une roue dentée centrale droite (12.2) comportant au moins une patte de retenue (12.2.2) retenant le pelliculage, et d'une troisième roue à ailettes (12.1), sur laquelle la couche protectrice du ruban d'emballage pelliculé est enroulée, et d'une quatrième roue dentée (12.3) entraînée par la second roue dentée (11.2).

5. Dispositif inhalateur de poudre sèche (7) suivant la revendication 1, **caractérisé en ce qu'**il comprend une patte de retenue du pelliculage (14.1), intégrée à ladite seconde roue dentée supplémentaire (14) et fixant l'extrémité de la couche principale de pelliculage à ladite seconde roue dentée supplémentaire (14).

6. Dispositif inhalateur de poudre sèche (7) suivant la revendication 1, **caractérisé en ce qu'**il comprend au moins un moyen de guidage (1.3), disposé sur la surface intérieure du boîtier extérieur (1), et au moins un élément de guidage (5.5) correspondant guidé par rapport au moyen de guidage (1.3), en étant situé sur la surface extérieure de l'élément d'actionnement (5), afin d'assurer la commande de trajet de course lorsque l'élément d'actionnement est déplacé dans la fente (1.4).

7. Dispositif inhalateur de poudre sèche (7) suivant la revendication 1, **caractérisé en ce qu'**il comprend une pièce d'ajustement de distance (1.1) située sur le boîtier extérieur (1) pour empêcher ledit élément d'actionnement (5) de sortir de la fente (1.4) pendant son déplacement de translation.

8. Dispositif inhalateur de poudre sèche (7) suivant la revendication 1, **caractérisé en ce qu'**il comprend au moins une surface de déplacement circulaire (8.3) de forme courbe sur la surface inférieure de l'élément éclipsable de verrouillage (8), servant à permettre à l'élément d'actionnement (5) de suivre un mouvement axial sur la surface inférieure de l'élément éclipsable de verrouillage (8).

9. Dispositif inhalateur de poudre sèche (7) suivant les revendications 1 et 4, **caractérisé en ce qu'**il comprend au moins un doigt de verrouillage (6.2) intégré à la partie intérieure du couvercle (6), servant à verrouiller le mécanisme de manoeuvre du dispositif (7) en s'engageant entre les dents de la quatrième roue dentée (12.3) lorsque le couvercle est fermé dans une mesure totale.

10. Dispositif inhalateur de poudre sèche (7) suivant les revendications 1 et 9, **caractérisé en ce qu'**il comprend au moins une ouverture (1.2) sur le boîtier extérieur (1), servant à permettre au doigt de verrouillage (6.2) de s'engager entre les dents de la quatrième roue dentée (12.3).

11. Dispositif inhalateur de poudre sèche (7) suivant la revendication 1, **caractérisé en ce que** le ressort (9) est en forme de V, ledit ressort étant mis en charge lorsque l'élément d'actionnement (5) est poussé dans la fente (1.4) et provoquant la sortie de l'élément d'actionnement (5) lorsque celui-là est relâché.
